# EUROPEAN PATENT SPECIFICATION

(11) **EP 2 175 861 B1**
(45) Date of publication and mention of the grant of the patent: **20.08.2014**
(21) Application number: 08778018.5
(22) Date of filing: 03.07.2008
(51) Int. Cl.: A61K 45/06, A61K 31/557, A61K 31/192, A61K 31/20, A61P 1/04, A61P 29/00, A61P 25/28, A61P 35/00

(54) **Pharmaceutical combination of naproxen with 13,14-dihydro-15-keto-16,16-difluoro-18-methyl-prostaglandin E1**
Pharmazeutische Kombination von Naproxen und 13,14-Dihydro-15-Keto-16,16-Difluor-18-Methyl-Prostaglandin E1
Combinaison pharmaceutique de naproxène et de 13,14-dihydro-15-céto-16,16-difluoro-18-méthyl-prostaglandine E1

(30) Priority: 03.07.2007 US 929557 P; 18.03.2008 US 37517
(43) Date of publication of application: 21.04.2010
(73) Proprietor: Sucampo AG, 6300 Zug (CH)
(72) Inventor: UENO, Ryuji, Montgomery, Maryland 20854 (US)
(74) Representative: Albrecht, Thomas
(86) International application number: PCT/JP2008/062437
(87) International publication number: WO 2009/005172

(56) References cited:
- WO-A-00/25771
- WO-A-00/56339
- WO-A-99/12524
- WO-A-2006/109881
- WO-A-2007/064664
- WO-A-2007/103540
- WO-A-2008/029949
- WO-A-2008/076703
- CA-A1- 1 041 012
- GB-A- 1 466 051
- US-A- 3 781 429
- US-A- 3 928 588
- US-B1- 6 265 440
- HIXSON L J ET AL: "CURRENT TRENDS IN THE PHARMACOTHERAPY FOR PEPTIC ULCER DISEASE" ARCHIVES OF INTERNAL MEDICINE, AMERICAN MEDICAL ASSOCIATION, CHICAGO, IL, US, vol. 152, no. 4, 1 January 1992 (1992-01-01), pages 726-732, XP008066895 ISSN: 0003-9926
- OSAMA H ET AL: "W1939 Effect of Cobiprostone On Naproxen-Induced Gastric Ulcers in Rats" GASTROENTEROLOGY, ELSEVIER, PHILADELPHIA, PA, vol. 134, no. 4, 1 April 2008 (2008-04-01), pages A-738, XP023435243 ISSN: 0016-5085 [retrieved on 2008-04-01]

## Description

### TECHNICAL FIELD

The present invention relates to a pharmaceutical combination of naproxen as a NSAID and a specific prostaglandin compound.

Non steroidal anti-inflammatory drugs (NSAIDs) are the most frequently prescribed drugs worldwide for the treatment of pain from various etiologies. Commonly used NSAIDs include sulindac, naproxen, indomethacin, mefenamic acid, diclofenac, fenoprofen, and diflunisal. Recently NSAIDs have been considered for treatment and prevention of Alzheimer's disease (Trends Pharmacol Sci. 28(10), 536-543, 2007). In addition, NSAIDs could reduce the risk of cancer through the inhibition of cyclooxygenage-2 (COX-2), and are shown a protective effect in colon, rectum, esophagus, stomach, pancreas, breast, prostate, lung and bladder cancer, suggesting a common mechanistic effect of NSAIDs in all these different cancers (BMC Cancer 2003, 3:28 and Annals of Internal Medicine 146(5), 376-389, 2007). Despite the therapeutic benefits of NSAIDs, their use is commonly limited by an increased risk of gastrointestinal(GI) side effects such as gastritis and gastric ulcer.

Prostaglandins (hereinafter, referred to as PGs) are members of class of organic carboxylic acids, which are contained in tissues or organs of human or other mammals, and exhibit a wide range of physiological activity. PGs found in nature (primary PGs) generally have a prostanoic acid skeleton as shown in the formula (A):

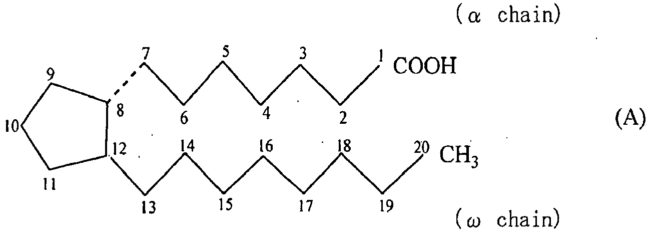

PGs are classified into several types according to the structure and substituents on the five-membered ring, for example,
Prostaglandins of the A series (PGAs); 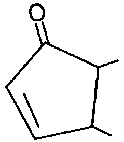 Prostaglandins of the B series (PGBs); 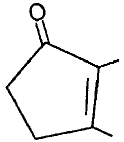 Prostaglandins of the C series (PGCs); 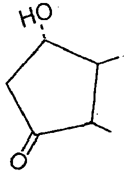 Prostaglandins of the D series (PGDs); 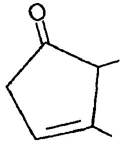 Prostaglandins of the E series (PGEs); 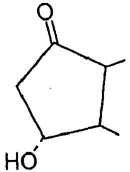 Prostaglandins of the F series (PGFs); 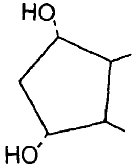 and the like. Further, they are classified into PG₁s containing a 13,14-double bond; PG₂s containing, 5,6- and 13,14-double bonds; and PG₃s containing 5,6-, 13,14- and 17,18-double bonds. PGs are known to have various pharmacological and physiological activities, for example, vasodilatation, inducing of inflammation, platelet aggregation, stimulating uterine muscle, stimulating intestinal muscular activity, anti-ulcer effects and the like.

U.S. Patent Nos. 5,225,439, 5,166,174, 5,284,858, 5,428,062, 5,380,709, 5,876,034 and 6,265,440 describe that certain prostaglandin E compounds are effective for the treatment of ulcers such as duodenal ulcer and gastric ulcer.

U.S. Patent No. 5,317,032 to Ueno et al. describes prostaglandin analog cathartics, including the existence of bicyclic tautomers and U.S. Patent No. 6,414,016 to Ueno describes the bicyclic tautomers as having pronounced activity as anti-constipation agents. The bicyclic tautomers, substituted by one or more halogen atoms can be employed in small doses for relieving constipation. At the C-16 position, especially, fluorine atoms, can be employed in small doses for relieving constipation.

U.S. Patent No. 7064148 to Ueno et al. describes prostaglandin compound opens and activates chloride channels, especially ClC channels, more especially ClC-2 channel.

U.S Patent publication No. 2003/0166632 to Ueno described ClC-2 channel opener is effective for the treatment of a disease or a condition responsive to opening of ClC-2 channel.

U.S. Patent publication No. 2003/0119898 to Ueno et al. describes specific composition of a halogenated prostaglandin analog for the treatment and prevention of constipation.

U.S. Patent publication No. 2004/0138308 to Ueno et al. describes chloride channel opener, especially a prostaglandin compound for the treatment of abdominal discomfort, and the treatment of functional gastrointestinal disorders such as irritable bowel syndrome and functional dyspepsia.

### DISCLOSURE OF THE INVENTION

The present invention relates to a pharmaceutical combination of:
(a) naproxen or a pharmaceutical salt thereof, and
(b) 13,14-dihydro-15-keto-16,16-difluoro-18-methyl-prostaglandin E₁.

By combining the NSAID and the PG compound of the present invention, the effect of the NSAID is augmented and/or the adverse side effect of the NSAID such as ulcer formation is well suppressed.

In another aspect of the invention, there is provided a pharmaceutical composition comprising the combination of the present invention in association with a pharmaceutically suitable excipient.

According to the present invention, the composition may be formulated as separate dosage forms each comprising individual active ingredient or as a single dosage form comprising the two active ingredients.

Further, the present invention provides the combination for use in treating a condition or disease which is one of the indications for NSAID use, which comprises administering a combination of the present invention.

According to the present invention, the (a) and (b) compounds may be administered simultaneously, separately or sequentially.

### DETAILED DESCRIPTION OF THE INVENTION

### (a) NSAID

The NSAID is naproxen.

### (b) The prostaglandin compound

The nomenclature of the prostaglandin compound used herein is based on the numbering system of the prostanoic acid represented in the above formula (A).

The formula (A) shows a basic skeleton of the C-20 carbon atoms. In the formula (A), the numbering of the carbon atoms which constitute the basic skeleton of the PG compounds starts at the carboxylic acid (numbered 1), and carbon atoms in the α-chain are numbered 2 to 7 towards the five-membered ring, those in the ring are 8 to 12, and those in the ω-chain are 13 to 20. Stereochemistry of the compounds is the same as that of the above formula (A) unless otherwise specified. Stereochemistry of PGs having hydroxy, lower alkyl or hydroxy(lower)alkyl substituent at position 9 and/or 11 may be α, β or a mixture thereof.

Suitable "pharmaceutically acceptable salts" include conventionally used non-toxic salts, for example a salt with an inorganic base such as an alkali metal salt (such as sodium salt and potassium salt), an alkaline earth metal salt (such as calcium salt and magnesium salt), an ammonium salt; or a salt with an organic base, for example, an amine salt (such as methylamine salt, dimethylamine salt, cyclohexylamine salt, benzylamine salt, piperidine salt, ethylenediamine salt, ethanolamine salt, diethanolamine salt, triethanolamine salt, tris(hydroxymethylamino)ethane salt, monomethyl- monoethanolamine salt, procaine salt and caffeine salt), a basic amino acid salt (such as arginine salt and lysine salt), tetraalkyl ammonium salt and the like. These salts may be prepared by a conventional process, for example from the corresponding acid and base or by salt interchange.

The prostaglandin compound is 13,14-dihydro-15-keto-16,16-difluoro-18-methyl-prostaglandin E₁ compound.

The configuration of the ring and the α- and/or ω chains in the above compound may be the same as or different from that of the primary PGs. However, the present invention also includes a mixture of a compound having a primary type configuration and a compound of a non-primary type configuration.

In the present invention, the PG compound which is dihydro between 13 and 14, and keto(=O) at 15 position may be in the keto-hemiacetal equilibrium by formation of a hemiacetal between hydroxy at position 11 and keto at position 15.

For, example, it has been revealed that when both of X₁ and X₂ are halogen atoms, especially, fluorine atoms, the compound contains a tautomeric isomer, bicyclic compound.

If such tautomeric isomers as above are present, the proportion of both tautomeric isomers varies with the structure of the rest of the molecule or the kind of the substituent present. Sometimes one isomer may predominantly be present in comparison with the other. However, it is to be appreciated that the present invention includes both isomers.

Further, the 15-keto-PG compound used in the invention includes the bicyclic compound.

Furthermore, while the compound used in the invention may be represented by a formula or name based on keto-type regardless of the presence or absence of the isomers, it is to be noted that such structure or name does not intend to exclude the hemiacetal type compound.

In the present invention, any of isomers such as the individual tautomeric isomers, the mixture thereof, or optical isomers, the mixture thereof, a racemic mixture, and other steric isomers may be used in the same purpose.

### The Pharmaceutically Suitable Excipient

According to the invention, the combination may be formulated in a pharmaceutical composition.. The composition may be formulated as separate dosage forms each comprising individual active ingredient with a pharmaceutically suitable excipient, or as a single dosage form comprising two active ingredients with a pharmaceutically suitable excipient. The pharmaceutically suitable excipient may be, therefore, selected depending on the desired form of the composition. According to the invention, "pharmaceutically suitable excipient" means an inert substance, which is suitable for the form, combined with the active ingredient of the invention.

For example, solid composition for oral administration of the present invention may include tablets, preparations, granules and the like. In such a solid composition, one or more active ingredients may be mixed with at least one inactive diluent, for example, lactose, mannitol, glucose, hydroxypropyl cellulose, microcrystalline cellulose, starch, polyvinyl pyrrolidone, magnesium aluminate metasilicate and the like. According to the usual work-up, the composition may contain additives other than inactive diluent, for example, lubricant such as magnesium stearate; disintegrant such as fibrous calcium gluconate; stabilizer such as cyclodextrin, for example, α, β- or γ-cyclodextrin; etherified cyclodextrin such as dimethyl-α-, dimethyl-β-, trimethyl-β-, or hydroxypropyl-β-cyclodextrin; branched cyclodextrin such as glucosyl-, maltosyl-cyclodextrin; formylated cyclodextrin, cyclodextrin containing sulfur; phospholipid and the like. When the above cyclodextrins are used, an inclusion compound with cyclodextrins may be sometimes formed to enhance stability. Alternatively, phospholipid may be sometimes used to form a liposome, resulting in enhanced stability.

Tablets or pills may be coated with film soluble in the stomach or intestine such as sugar, gelatin, hydroxypropyl cellulose, or hydroxypropylmethyl cellulose phthalate as needed. Further, they may be formed as capsules with absorbable substances such as gelatins. Preferably, the composition is formulated in a soft gelatin capsule with liquid contents of the specific prostaglandin compound and a medium chain fatty acid triglyceride. Examples of the medium chain fatty acid triglyceride used in the present invention include a triglyceride of a saturated or unsaturated fatty acid having 6-14 carbon atoms which may have a branched chain. A preferred fatty acid is a straight chain saturated fatty acid, for example caproic acide (C6), caprylic acid (C8), capric acid (C10), lauric acid (C12) and myristic acid (C14). In addition, two or more medium chain fatty acid triglycerides may be used in combination. Further suitable excipients are disclosed in the published PCT application WO 01/27099.

A liquid composition for oral administration may be pharmaceutically acceptable emulsion, solution, suspension, syrup, or elixir, as well as generally used inactive diluent. Such composition may contain, in addition to the inactive diluent, adjuvants such as lubricants and suspensions, sweetening agents, flavoring agents, preservatives, solubilizers, anti-oxidants and the like. The details of the additives may be selected from those described in any general textbooks in the pharmaceutical field. Such liquid compositions may be directly enclosed in soft capsules. Solutions for parenteral administration, for example, suppository, enema and the like according to the present invention include sterile, aqueous or non-aqueous solution, suspension, emulsion, detergent and the like. The aqueous solution and suspension includes, for example, distilled water, physiological saline and Ringer's solution.

The non-aqueous solution and suspension include, for example, propylene glycol, polyethylene glycol, fatty acid triglyceride, and vegetable oil such as olive oil, alcohols such as ethanol, polysorbate and the like. Such composition may contain adjuvants such as preservatives, wetting agent, emulsifier, dispersant, anti-oxidants and the like.

Examples of the injectable compositions of the present invention for parenteral administration include sterile aqueous or non-aqueous solutions, suspensions and emulsions. Diluents for the aqueous solution or suspension may include, for example, distilled water for injection, physiological saline and Ringer's solution.

Non-aqueous diluents for solution and suspension may include, for example, propylene glycol, polyethylene glycol, vegetable oils such as olive oil, alcohols such as ethanol and polysorbate. The composition may further comprise additives such as preservatives, wetting agents, emulsifying agents, dispersing agents and the like. They may be sterilized by filtration through, e.g. a bacteria-retaining filter, compounding with a sterilizer, or by means of gas or radioisotope irradiation sterilization. The injectable composition may also be provided as a sterilized powder composition to be dissolved in a sterilized solvent for injection before use.

Another form of the present invention is suppository or pessary, which may be prepared by mixing active ingredients into a conventional base such as cacao butter that softens at body temperature, and nonionic surfactants having suitable softening temperatures may be used to improve absorbability.

According to the method of the invention, the combination of the present invention can be administered systemically or locally by means of oral or parental administration, including a suppository, enema and the like. Single or multiple compositions may be administered to achieve the desired dose. According to the method, individual compounds in the combination may be administered simultaneously, separately, or sequentially.

According to the present invention, a mammalian subject may be treated by the instant invention by administering the compound used in the present invention. The mammalian subject may be any subject including a human. The compound may be applied systemically or topically. Usually, the compound may be administered by oral administration, intravenous injection (including infusion), subcutaneous injection, intra rectal administration, intra vaginal administration, transdermal administration and the like. The dose may vary depending on the strain of the animal, age, body weight, symptom to be treated, desired therapeutic effect, administration route, term of treatment and the like. A satisfactory effect can be obtained by systemic administration 1-4 times per day or continuous administration at combination with the amount of 0.001-100000 µg, more preferably 0.01-10000 µg, especially 0.1-1000 µg of specific prostaglandin compound, and 0.01-100000mg, more preferably 0.1-10000mg, of NSAID at a daily dose.

The term "combination" used herein means naproxen (NSAID) and the specific prostaglandin compound are both administered to a patient simultaneously in the form of a single entity or dosage, or are both administered to a patient as separate entities either simultaneously or sequentially with no specific time limits, wherein such administration provides therapeutically effective levels of the two components in the body, preferably at the same time.

The combination of the present invention is useful for the treatment of a disease or condition which is one of the indications for NSAID use. For example, the combination is useful for the treatment of arthritis, rheumatoid arthritis; osteoarthritis, spondylitis, ankylosing spondylitis, juvenile arthritis, juvenile rheumatoid arthritis, tendonitis, bursitis, gout, pain, and dysmenorrhea.

The term "treatment" used herein includes any means of control such as prevention, care, relief of the condition, attenuation of the condition and arrest of progression.

The combination of the present invention is useful for the treatment of pain from various etiologies.

The term "pain from various etiology" includes, but is not limited to, inflammatory pain, hyperalgesia and, in particular, chronic pain, and means in particular pain consequential to trauma, e.g. associated with burns, sprains, fracture or the like, subsequent to surgical intervention, e.g. as post-operative analgesics, chemotherapy-induced pain, as well as inflammatory pain of diverse genesis, e.g. bone and joint pain (osteoarthritis), myofascial pain (muscular injury, fibromyalgia), lower back pain, chronic inflammatory pain, chronic neuropathic pain, e.g. diabetic neuropathy, phantom limb pain and perioperative pain (general surgery, gynecologic surgery) as well as pain associated with, e.g., angina, menstruation or cancer.

The further details of the present invention will follow with reference to test examples, which, however, are not intended to limit the present invention.

### Example 1

Male Crl: CD (SD) rats were orally administered with naproxen at 200 mg/kg in combination with Compound A (13,14-dihydro-15-keto-16,16-difluoro-18(S)-methyl-PGE₁ or vehicle. The animal was euthanized by cervical dislocation 5 hours after administration of the dosing preparation, and the stomach was removed. The stomach was filled with 10 mL of saline and fixed in 1% formalin. Then the stomach was opened along the greater curvature and gently washed with saline. The length of the major axis of each gastric ulcer was measured with digital slide gauges, and the total length of the major axes of all gastric ulcers (ulcer index) was obtained.

As shown in Table 1, the ulcer formation induced by naproxen at 200 mg/kg was reduced by the combined administration of Compound A at 1, 10 and 100 µg/kg with naproxen (200 mg/kg) in a dose-dependent manner. Significant reductions of ulcer formation were observed in the " Compound A (10 µg/kg) + naproxen (200 mg/kg) group" and "Compound A (100 µg/kg) + naproxen (200 mg/kg) group" compared to "Vehicle + naproxen (200 mg/kg) group". The combined administration of lansoprazole, a proton pump inhibitor used for the treatment of gastric ulcer in clinic, at 1000 µg/kg with naproxen did not have a significant effect on the naproxen-induced gastric ulcers.

The results indicate that Compound A has a potent protective effect against ulcer formation in combination with non-steroidal anti-inflammatory drug (NSAID).

**Table 1 Effect of Compound A in combination with naproxen on naproxen-induced gastric ulcer in rats**

| Group | n | Ulcer Index (Mean ± S.E.) | %Inhibition^{a} |
|---|---|---|---|
| Vehicle (0.01% Tween 80) + naproxen (200 mg/kg) | 10 | 81.48 ± 15.05 | - |
| Compound A (1 µg/kg) + naproxen (200 mg/kg) | 10 | 63.47 ± 10.87 | 22% |
| Compound A (10 µg/kg) + naproxen (200 mg/kg) | 10 | 12.15 ± 3.45* | 85% |
| Compound A (100 µg/kg) + naproxen (200 mg/kg) | 10 | 0.50 ± 0.40** | 99.4% |
| Lansoprazole (1000 µg/kg) + naproxen (200 mg/kg) | 10 | 68.09 ± 7.61 | 16% |

| | | | |
|---|---|---|---|
| * p<0.05, ** p<0.01 compared to "vehicle + naproxen group" (Dunnett-type test) a Calculated as: % inhibition =(1-mean ulcer index in test group/mean ulcer index in control group)X 100 | | | |

## Claims

1. A pharmaceutical combination of:
(a) naproxen or a pharmaceutically acceptable salt thereof, and
(b) 13,14-dihydro-15-keto-16,16-difluoro-18-methyl-prostaglandin E₁.

2. The combination as described in Claim 1 for use in the treatment of a condition or a disease which is one of the indications for NSAID use selected from the group consisting of pain, inflammatory pain, hyperalgesia, chronic pain, pain consequential to trauma, pain subsequent to surgical intervention, chemotherapy-induced pain, inflammatory pain of diverse genesis, bone and joint pain, osteoarthritis, myofascial pain, lower back pain, chronic inflammatory pain, chronic neuropathic pain, diabetic neuropathy, phantom limb pain, perioperative pain, pain associated with angina, menstruation or cancer.

3. The combination for use as described in Claim 2, wherein the condition or disease is pain.

4. The combination for use as described in Claim 2, wherein the condition or disease is chronic pain.

5. The combination as described in Claim 1 for use as a medicament for administering the respective compounds simultaneously, separately or sequentially.

6. A pharmaceutical composition comprising the combination as described in Claim 1 in association with a pharmaceutically suitable excipient.

7. The composition as described in claim 6, wherein said pharmaceutically suitable excipient is orally acceptable.

8. The composition as described in claim 6, wherein the composition is formulated as separate dosage forms each comprising individual active agent or as a single dosage form comprising the two active ingredients.

## Patentansprüche

1. Pharmazeutische Kombination aus:
(a) Naproxen oder einem pharmazeutisch annehmbaren Salz davon und
(b) 13,14-Dihydro-15-keto-16, 16-difluor-18-methyl-prostaglandin E₁.

2. Kombination nach Anspruch 1 zur Verwendung bei der Behandlung eines Zustands oder einer Erkrankung, bei denen es sich um eine Indikation für die Verwendung von NSARs (nichtsteroidale Antirheumatika) handelt, ausgewählt aus der Gruppe, bestehend aus Schmerzen, entzündungsbedingten Schmerzen, Hyperalgesie, chronischen Schmerzen, Schmerzen infolge von Trauma, Schmerzen infolge von chirurgischer Intervention, chemotherapiebedingten Schmerzen, entzündungsbedingten Schmerzen diverser Ursachen, Knochen- und Gelenkschmerzen, Osteoarthritis, Myofaszialschmerzen, Kreuzschmerzen, chronischen entzündungsbedingten Schmerzen, chronischen neuropathischen Schmerzen, diabetischer Neuropathie, Phantom-Gliedmaßenschmerzen, perioperativen Schmerzen, Schmerzen, die mit Angina, Menstruation oder Krebs assoziiert sind.

3. Kombination zur Verwendung nach Anspruch 2, wobei der Zustand oder die Erkrankung Schmerzen ist.

4. Kombination zur Verwendung nach Anspruch 2, wobei der Zustand oder die Erkrankung chronische Schmerzen ist.

5. Kombination nach Anspruch 1 zur Verwendung als Medikament zur gleichzeitigen, separaten oder sequentiellen Verabreichung der jeweiligen Verbindungen.

6. Pharmazeutische Zusammensetzung, umfassend die Kombination nach Anspruch 1 in Verbindung mit einem pharmazeutisch geeigneten Hilfsstoff.

7. Zusammensetzung nach Anspruch 6, wobei der pharmazeutisch geeignete Hilfsstoff oral annehmbar ist.

8. Zusammensetzung nach Anspruch 6, wobei die Zusammensetzung formuliert ist als separate Dosierungsformen, die jeweils einzelne aktive Mittel umfassen, oder als einzelne Dosierungsform, die die beiden aktiven Bestandteile umfasst.

## Revendications

1. Combinaison pharmaceutique :
(a) de naproxène ou d'un sel pharmaceutiquement acceptable de celui-ci, et
(b) de 13,14-dihydro-15-céto-16,16-difluoro-18-méthyl-prostaglandine E₁.

2. Combinaison selon la revendication 1 pour utilisation dans le traitement d'un état ou d'une maladie qui est l'une des indications pour l'utilisation d'un AINS, choisi dans le groupe constitué par une douleur, une douleur inflammatoire, une hyperalgésie, une douleur chronique, une douleur consécutive à un traumatisme, une douleur consécutive à une intervention chirurgicale, une douleur induite par une chimiothérapie, une douleur inflammatoire d'origine diverse, une douleur osseuse et articulaire, l'arthrose, une douleur myofasciale, une douleur du bas du dos, une douleur inflammatoire chronique, une douleur neuropathique chronique, une neuropathie diabétique, une douleur d'un membre fantôme, une douleur périopératoire, une douleur associée à une angine, aux menstruations, ou à un cancer.

3. Combinaison pour utilisation selon la revendication 2, dans laquelle l'état ou la maladie est une douleur.

4. Combinaison pour utilisation selon la revendication 2, dans laquelle l'état ou la maladie est une douleur chronique.

5. Combinaison selon la revendication 1, pour utilisation en tant que médicament destiné à l'administration des composés respectifs simultanément, séparément ou successivement.

6. Composition pharmaceutique comprenant la combinaison selon la revendication 1 en association avec un excipient pharmaceutiquement convenable.

7. Composition selon la revendication 6, dans laquelle ledit excipient pharmaceutiquement convenable est acceptable par voie orale.

8. Composition selon la revendication 6, laquelle composition est formulée sous des formes galéniques distinctes comprenant chacune un agent actif individuel ou sous une forme galénique unique comprenant les deux ingrédients actifs.
